# EUROPÄISCHE PATENTANMELDUNG

(11) **EP 1 679 062 A1**
(43) Veröffentlichungstag der Anmeldung: **12.07.2006**
(21) Anmeldenummer: 05024373.2
(22) Anmeldetag: 09.11.2005
(51) Int. Cl.: A61K 8/46, A61K 8/81, A61Q 19/10, A61K 8/31, A61K 8/37, A61Q 5/02

(54) **Tensidhaltige Emulsionen für das Wannenbad mit hoher Badewassertrübung**

(30) Priorität: 10.01.2005 DE 102005001785
(71) Anmelder: Beiersdorf AG, 20253 Hamburg (DE)
(72) Erfinder: Kohut, Michaela, 20257 Hamburg (DE); Ruppert, Stephan, 20259 Hamburg (DE); Küther, Jörg, 25469 Halstenbek (DE); Liste, Kathrin, 22301 Hamburg (DE)

(57) **Zusammenfassung**

Kosmetische Reinigungsemulsion enthaltend:
a) 41 - 51 %, besonders bevorzugt 43-47% Ölphase,
b) 2 - 17%, besonders bevorzugt 6-12% eines anionischen Tensids und
c) 0,1 bis 0,6%, besonders bevorzugt 0,3-0,6% eines Polyacrylates enthält,
wobei das Gewichtsverhältnis der Gesamtmenge Polyacrylat und der Gesamtmenge an Ölen vorteilhaft im Verhältnis von von 1:135 bis 1:85 und bevorzugt von 1:115 bis 1:95 gewählt wird und die Zubereitung eine Viskosität von 250 - 500 mPas bei 100 1/s aufweist, gemessen mit Ares 8 aus der Rheometrix-Serie der Firma TA Instruments bei 25°C in einem DIN Zylinder-System (Kegel-Platte System mit einem Durchmesser von 50mm) bei einer Scherrate von 100 1/s.

## Beschreibung

Die vorliegende Erfindung betrifft tensidhaltige Emulsionen für das Wannenbad mit hoher Badewassertrübung.

In der Kosmetik sind bisher verschiedene Formen von Badezusätzen bekannt. Dazu zählen die klassischen Ölbäder, mehrphasige Zubereitungen wie Schaumbäder, auch in Form von Zubereitungen, die in Ruhe erkennbar zweiphasig sind und durch Schütteln homogenisiert werden können, sich aber nach einiger Zeit wieder trennen. Darüber hinaus gibt es Badesalze, also feste Zubereitungen, die beim Badewasser zugesetzt und aufgelöst werden müssen.

All diese Produkte weisen Nachteile auf:
Ölbäder sind in der Regel sehr flüssig und weisen oft Mängel bei der Schaumentwicklung auf. Ölbäder weisen einen geringen Tensidgehalt auf. Daher bildet sich auf der Oberfläche des Badewassers ein Ölfilm. Dieser vermittelt zwar dem Badenden ein Pflegegefühl, das Öl zieht allerdings erst beim Aussteigen aus dem Badewasser auf die Haut auf und bildet einen Film auf der Haut des Aussteigende. Dieser Film wird meist direkt ins Handtuch hineingerieben. Der Ölfilm überzieht während des Ablassen des Badewassers die Badewanne und trocknet kaum sichtbar an, wodurch für den nächsten Benutzer ein erhebliches Risiko des Ausrutschens beim Betreten der Wanne entsteht. Der Ölrückstand ist darüber hinaus sichtbar. Daher muß er später entfernt werden, beispielsweise durch Einsatz weiterer Reinigungsmittel. Diese belasten das Abwasser zusätzlich.

Darüber hinaus können Ölbäder nicht in beliebigen Farben eingefärbt werden, da es nur wenige für Kosmetika verwendbare Farbstoffen gibt. Deren Farben violett, grün, rot und Mischfarben davon wirken jedoch zuweilen etwas altbacken.

Schaumbäder weisen eher geringe Ölanteile von 0, 1 % bis 2%. auf. Schaumbäder weisen aufgrund ihres hohen Wasseranteils eine gute Löslichkeit und aufgrund des geringen Ölanteils sehr gute Schaumeigenschaften auf. Der geringe Ölanteil ist Ursache dafür, das solche Produkte der gebadeten Haut nur wenig Pflege vermitteln. Der Anwender muss daher oftmals auf ein seperates Pflegeprodukt (leave on) zurückgreifen. Darüber hinaus führen solche Schaumbäder nur zu einer geringen Trübung des Badewassers. Eine starke Trübung des Badewassers ist aber erwünscht, um dem Badenden auch visuell die Anmutung besonderer Pflege zu wecken, wie sie von einem milchig-trüben Badewasser ausgeht. Auch die Assoziation von cremiger Pflege kann kaum von nahezu klarem Badewasser vermittelt werden.

Auch der Einsatz moderner Duschprodukte in der Badewanne führt zu unbefriedigenden Ergebnissen. Die meisten Duschgele, Duschcremes und auch Duschöl vermischen sich nicht genügend schnell mit dem Badewasser, so dass sich unschöne Würste aus nicht aufgelöstem Produkt auf dem Boden der Badewanne ansammeln. Dadurch müssen meist erheblich höhere Mengen Produkt dem Badewasser zugesetzt werden. Duschgele führen darüber hinaus zu nahezu ungetrübtem Badewasser. Weiterhin führt der Einsatz von tensidfreien Emulsionen zu äußerst geringer bis gar keiner Schaumbildung, wenn sie wie üblich dem Badewasser zugesetzt werden.

Duschöl bildet einen Ölfilm wie ein Ölbad. Alle diese Produkte bilden nur widerwillig Schaum mit der erforderlichen, gegenüber Duschprodukten höheren Stabilität gegen den Zerfall.

EP 1166772 offenbart kosmetische oder dermatologische Reinigungsemulsionen, die bezogen auf das Gesamtgewicht der Zubereitungen 1 bis 30 Gew.-% eines oder mehrerer waschaktiver Tenside, gewählt aus der Gruppe der Tenside, welchen einen HLB-Wert von mehr als 15 haben, 35 bis 50 Gew.-% einer oder mehrerer Ölkomponenten, 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und 5 bis 60 Gew.-% Wasser enthalten.

DE 10153023 offenbart Reinigungsemulsionen, die bezogen auf das Gesamtgewicht der Zubereitungen 1 bis 30 Gew.-% eines oder mehrerer waschaktiver Tenside, gewählt aus der Gruppe der Tenside, welchen einen HLB-Wert von mehr als 15 haben, 35 bis 50 Gew.-% einer oder mehrerer Ölkomponenten, 0,001 bis 30 Gew.-% eines oder mehrerer Wirkstoffe, 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und 5 bis 60 Gew.-% Wasser enthalten.

DE 10161171 offenbart kosmetische oder dermatologische Reinigungsemulsionen, die bezogen auf das Gesamtgewicht der Zubereitungen 1 bis 30 Gew.-% eines oder mehrerer waschaktiver Tenside, gewählt aus der Gruppe der Tenside, welchen einen HLB-Wert von mehr als 15 haben, 35 bis 50 Gew.-% einer oder mehrerer Ölkomponenten, 0,1 bis 10 Gew.-% Talkum, 0,2 bis 5 Gew.-% eines oder mehrerer Polyacrylate, gewählt aus der Gruppe, welche gebildet wird aus anionischen Homo- und/oder Copolymeren der Acrylsäure und/oder alkylierten Acrylsäurederivaten sowie deren Estern und 5 bis 60 Gew.-% Wasser enthalten.

EP 0820272 offenbart Zusammensetzungen in Form von O/W-Emulsionen, die frei von bestimmten 1-Alkenen sind und aus 0,01 bis 5 Gew.% bestimmter Copolymere von beispielsweise Acrylsäure, 0,05 bis 20 Gew.% eines nichtemulgiernden Waqschtensides mit einem HLB-Wert grösser 11, 0,05 bis 40 Gew.% bestimmter Öle und 20 bis 99,44 Gew.% Wasser.

Ausgehend von all dem fehlt es dem Stand der Technik an einem Produkt, das gute Schaumeigenschaften, gutes Auflöseverhalten und sehr gute Rückfettungseigenschaften bei guter Reinigungsleistung aufweist.

Es wurde nun gefunden, dass eine kosmetische Reinigungsemulsion enthaltend:
a) 41 - 51 %, besonders bevorzugt 43-47% Ölphase,
b) 2 - 17%, besonders bevorzugt 6-12% eines anionischen Tensids und
c) 0,1 bis 0,6%, besonders bevorzugt 0,3-0,6% eines Polyacrylates,
wobei das Gewichtsverhältnis der Gesamtmenge Polyacrylat und der Gesamtmenge an Ölen vorteilhaft im Verhältnis von von 1:135 bis 1:85 und bevorzugt von 1:115 bis 1:95 gewählt wird und die Zubereitung eine Viskosität von 250 - 500 mPas bei 100 1/s aufweist, gemessen mit Ares 8 aus der Rheometrix-Serie der Firma TA Instruments bei 25°C in einem DIN Zylinder-System (Kegel-Platte System mit einem Durchmesser von 50mm) bei einer Scherrate von 100 1/s den Mängeln des Standes der Technik abhilft.

Durch Entwicklung dieser speziellen Formulierungen gelingt es , ein Produkt zur Verfügung zu stellen, das gute Schaumeigenschaften, gutes Auflöseverhalten aufweist und sehr gute Rückfettungseigenschaften durch den hohen Ölgehalt sicherstellt.

Hier wurde eine langzeitstabile tensidhaltige Badeemulsion mit hohem Lipidgehalt generiert, die sich besonders gut unter Anwendungsbedingungen auflöst und ein gutes Anschäumverhalten zeigt. Trotz des sehr hohen Lipidanteils zeigt die Formulierung hervorragende Schaumeigenschaften (Schaummenge und -stabilität), die so nicht zu erwarten waren.

Eine solche Emulsion weist darüber hinaus eine sehr gute Duftentwicklung auf. Durch die Emulsionsstruktur und dadurch, dass die Formel im Badewasser nicht deemulgiert, ist man in der Lage die Ausrutschgefahr in der Badewanne zu vermindern. Außerdem bleiben kaum Ölränder in der Badewanne zurück, die sich nur schwer wieder entfernen lassen.

Das Produkt hat durch die Emulsionstruktur eine schöne kosmetische Anmutung und das ohne zusätzlichen Einsatz von Farbstoffen, Perlglanzstoffen oder Trübungsmittel.

Es wurde weiter gefunden, daß es bevorzugt ist, wenn eine solche Zubereitung im Wasser nicht deemulgiert. Weiter ist es bevorzugt, wenn der Trübungsindex einer solchen Zubereitung kleiner 10%, besonders bevorzugt kleiner 5% ist. Zur Bestimmung des Trübungsindex wird folgendermaßen vorgegangen: Das Produkt wird im Verhältnis von 1:5000 und 1:3000 mit 38°C warmem Wasser verdünnt. Danach erfolgt die Messung der Transmission mit dem UV-Spekrotmeter 8453 der Firma HP in einer 1 cm Küvette (Plastikküvette der Firma Plastibrand, Katalog-Nr. 759170) bei einer Wellenlänge von 420 nm. Der Trübungsindex gibt die Transmission in % bei einer Wellenlänge von 420 nm bei einer Verdünnung von 1:5000 an. Weiter bevorzugt ist es, wenn das Mindestschaumvolumen nach der Ross-Miles Methode bei 100 - 140 ml liegt, besonders bevorzugt 110 - 130 ml. Dabei wird folgendermaßen nach einer modifizierten Variante des Ross-Miles Testes (DIN 53 902) vorgegangen. Diese Abwandlung des Testes wurde von Dr. F.J. Gohlke (Hoechst AG, Frankfurt) in der Zeitschrift Parfümerie und Kosmetik Nr.3/1964, Seiten 59 - 63 beschrieben. Hierbei wird die Versuchsanordnung leicht modifiziert, wodurch sich im Vergleich zur DIN-Vorschrift etwas niedrigere Schaumhöhen ergeben.

Die Modifizierung sieht folgenden Versuchsaufbau vor:
50 ml der zu testenden Tensidlösung, auf 38° temperiert, werden in einen Standzylinder vorgelegt. Aus einer definierten Höhe wird 200 ml Wasser, ebenfalls auf 38° temperiert, mit einem Zusatz von 300 mg/l Calciumsulfat auf die Tensidlösung fallen gelassen. Die hierdurch erzeugte Schaummenge wird sofort, nach 30 Sekunden und nach 1, 3, 5, 10 bzw. 15 Minuten abgelesen und in ein Diagramm eingetragen. Aus den aufgenommenen Daten wird zusätzlich die Schaumstabilität errechnet und ebenfalls grafisch aufgetragen.

Bevorzugt ist es wenn als Polyacrylat ein oder mehrere Polyacrylate, welche gewählt werden aus der Gruppe, die gebildet wird aus anionischen Homo- und/oder Copolymeren der Acrylsäure und/oder alkylierten Acrylsäurederivaten sowie deren Estern, verwendet wird.
Ebenso bevorzugt ist es, als Polyacrylat ein oder mehrere Polyacrylate, welche gewählt werden aus der Gruppe, die gebildet wird aus anionischen Copolymeren der Acrylsäure und/oder alkylierten Acrylsäurederivaten sowie deren Estern, verwendet wird.
Erfindungsgemäß bevorzugt sind Acrylat-Copolymere und/oder Acrylat-Alkylacrylat-Copolymere, welche unter den Handelbezeichnungen Carbopol. 1382, Carbopol. 981 und Carbopol. 5984, Aqua SF-1 von der NOVEON Inc. bzw. als Aculyn. 33 von International Specialty Products Corp. erhältlich sind. Erfindungsgemäß besonders bevorzugt sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit. Vorteilhaft sind Verbindungen, die die lNCl-Bezeichnung "Acrylates/C 10-30 Alkyl Acrylate Crosspolymer" tragen. Insbesondere vorteilhaft sind die unter den Handelsbezeichnungen Carbopol ETD 2020, Carbopol 3128, Pemulen TR1 und Pemulen TR2 bei der NOVEON Inc. erhältlichen.
Besonders bevorzugt ist es, wenn das Polyacrylat quervernetzt ist und ganz besonders bevorzugt mit einem C10-C30-Alkylrest
Weiter ist es besonders bevorzugt, wenn die erfindungsgemässe Zubereitung ein oder mehrere Öle aus der Gruppe der Kohlenwasserstoffe, der Fettsäuretriglyceride, Carbonsäureester enthält. Dabei ist es besonders bevorzugt wenn das Verhältnis von Kohlenwasserstoffen zu Triglyceriden von 6:1 bis 2:1 und besonders bevorzugt von 5:1 bis 3:1 liegt. Dadurch wird die Spreitung und Rückfettung besonders vorteilhaft eingestellt. Weiter bevorzugt ist es, wenn das anionische Tensid Natriumlaurethsulfat, Natriumparethsulfat und/oder Natriummyrethsulfat oder Mischungen aus den vorgenannten Substanzen ist. Die Erfindung umfasst auch die Verwendung von solchen Zubereitungen zur Reinigung und Pflege von menschlicher oder animalischer Haut oder Haar und insbesondere die Verwendung als Wannenbad.

Ferner ist es erfindungsgemäß bevorzugt, als Polyacrylat Carbopol ETD 2020 von Noveon einzusetzen. Dieses weist die folgende Produkt Spezifikationen auf: Viskosität bei einem pH Wert von 5,8-6,3, bei 1% Einsatzkonzentration in Wasser, Brookfield RVT, 20 rpm bei 25°C 47000 - 77000 mPa s. Vorteilhaft ist es auch, wenn das eingesetzte Polyacrylat bei einer Einsatzkonzentration von 1 % und einem pH Wert von 5,2-5,6 in einer 10%igen wässrigen Natrium Laurylethersulfatlösung bei 25°C eine Viskosität von 500 - 1000 mPa s bei einer Scherrate von 100 1/s besitzt.

Die Zusammensetzungen enthalten gemäß der Erfindung außer den vorgenannten Substanzen gegebenenfalls die in der Kosmetik üblichen Zusatzstoffe, beispielsweise Parfüm, Farbstoffe, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Pigmente, die eine färbende Wirkung haben, Verdickungsmittel, weichmachende, anfeuchtende und/oder feuchthaltende Substanzen, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Erfindungsgemäße Zubereitungen können zusätzlich verkapselte Wirkstoffe oder Parfümöle enthalten.

Als Schaumverstärker können Tenside verwendet werden:
Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im allgemeinen nach Art und Ladung des hydrophilen Molekülteils klassifiziert. Hierbei können vier Gruppen unterschieden werden:
- anionische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quarternären Ammoniumgruppe gekennzeichnet. In wäßriger Lösung bilden sie im sauren oder neutralen Milieu positiv geladene organische lonen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wäßrigem Medium keine Ionen.
A. Anionische Tenside
   Vorteilhaft zu verwendende anionische Tenside sind
   Sulfonsäuren und Salze, wie
   1. Acyl-isethionate, z.B. Natrium-/ Ammoniumcocoyl-isethionat,
   2. Alkylarylsulfonate,
   3. Alkylsulfonate, beispielsweise Natriumcocosmonoglyceridsulfat, Natrium C₁₂₋₁₄ Olefin-sulfonat, Natriumlaurylsulfoacetat und Magnesium PEG-3 Cocamidsulfat,
   4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.
   sowie
   Schwefelsäureester, wie
   1. Alkylethersulfat, beispielsweise Natrium-, Ammonium-, Magnesium-, MIPA-, TIPA-Laurethsulfat, Natriummyrethsulfat und Natrium C₁₂₋₁₃-Parethsulfat,
   2. Alkylsulfate, beispielsweise Natrium-, Ammonium- und TEA-Laurylsulfat.
B. Amphotere Tenside
   Vorteilhaft zu verwendende amphotere Tenside sind Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
C. Nicht-ionische Tenside
   Vorteilhaft zu verwendende nicht-ionische Tenside sind
   1. Alkohole,
   2. Alkanolamide, wie Cocamide MEA/ DEA/ MIPA,
   3. Ester, die durch Veresterung von Carbonsäuren mit Ethylenoxid, Glycerin, Sorbitan oder anderen Alkoholen entstehen,
   4. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.

Vorteilhaft ist ferner die Verwendung einer Kombination von anionischen und/oder amphoteren Tensiden mit einem oder mehreren nicht-ionischen Tensiden.

Eine gegebenenfalls gewünschte Ölkomponente der kosmetischen oder dermatologischen Reinigungszubereitungen - beispielsweise in Form von Reinigungsemulsionen - im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, lsononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölkomponente vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 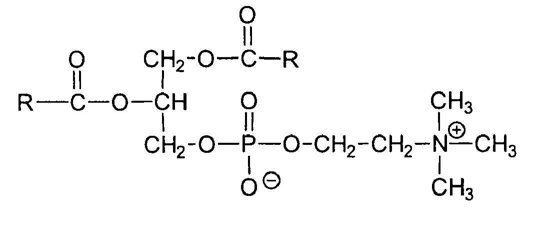 auszeichnen, wobei R' und R" typischerweise unverzweigte aliphatische Reste mit 15 oder 17 Kohlenstoffatomen und bis zu 4 cis-Doppelbindungen darstellen.

Vorteilhaft liegen Reinigungszubereitungen gemäß der Erfindung in Form von Gelen vor und enthalten einen oder mehrere Gelbildner bzw. Hydrokolloide.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

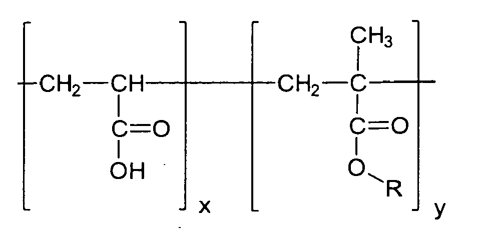

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCl-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCl-Bezeichnung "acrylates/C12-24 pareth-25 acrylate copolymer" (unter der Handelsbezeichnungen Synthalen® W2000 bei der 3V Inc. erhältlich), die die lNCl-Bezeichnung "acrylates/steareth-20 methacrylate copolymer" (unter der Handelsbezeichnungen Aculyn® 22 bei der International Specialty Products Corp. erhältlich), die die INCl-Bezeichnung "acrylates/steareth-20 itaconate copolymer" (unter der Handelsbezeichnungen Structure 2001 ® bei der National Starch erhältlich), die die lNCl-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen (Filmbildner). Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen in Shampoos stellen die polymeren quatären Ammoniumverbindungen dar. Auch können kationische Cellulosederivate und Polysaccharide eingesetzt werden. Weiterhin werden auch Silikonverbindungen zur Konditionierung eingesetzt.

Erfindungsgemäß vorteilhafte Filmbildner sind Polyquaternium-7, Polyquaternium-10, Polyquaternium-22 und Polyquaternium-44.

Ein Erfindungsgemäß besonders bevorzugter Filmbildner stellt Polyquarternium-10 (Ucare Polymer JR-125®, Ucare Polymer JR-400® von Amerchol) dar.

Weitere erfindungsgemäß vorteilhafte Filmbildner stellen Cellulosederivate und quaternisierte Guar Gum Derivate, insbesondere Guar Hydroxypropylammoniumchlorid (z.B. Jaguar Excel®, Jaguar C 162® der Firma Rhodia, CAS 65497-29-2, CAS 39421-75-5) dar.

Auch nichtionische Poly-N-vinylpyrrolidon/Polyvinylacetat-Copolymere (z.B. Luviskol VA 64W®, BASF), anionische Acrylat-Copolymere (z.B. Luviflex soft®, BASF), und/oder amphotere Amid/Acrylat/Methacrylat Copolymere (z.B. Amphomer®, National Starch) können erfindungsgemäß vorteilhaft als Filmbildner eingesetzt werden.

Es ist auch von Vorteil, den erfindungsgemäßen Zubereitungen Komplexbildner zuzusetzen. Vorteilhaft werden die Komplexbildner gewählt aus der Gruppe bestehend aus Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen, Tetrasodium Iminodisuccinate, Trisodium Etylenediamine Disuccinate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Es ist auch von Vorteil, den erfindungsgemäßen Zubereitungen Antioxidantien zuzusetzen. Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α -Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO4) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Emulsionen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Weiter ist es von Vorteil, Rückfetter in erfindungsgemäßen Zubereitungen einzusetzen:
Ethoxylierte Glycerin-Fettsäureester werden in wässrigen Reinigungsrezepturen zu verschiedenen Zwecken eingesetzt. Niedrig ethoxylierte Glycerin-Fettsäureester (EO 3-12) dienen üblicherweise als Rückfetter zur Verbesserung des Hautgefühls nach dem Abtrocknen, Glycerin-Fettsäureester mit einem Ethoxylierungsgrad von ca. 30-50 dienen als Lösungsvermittler für unpolare Substanzen wie Parfumöle. Hochethoxylierte Glycerin-Fettsäureester werden als Verdicker eingesetzt. Allen diesen Substanzen ist gemeinsam, dass sie auf der Haut bei der Anwendung i.e. bei der Verdünnung mit Wasser ein besonderes Hautgefühl erzeugen. Neuartig ist, dass diese Substanzen sowohl die inakzeptable Sensorik beim Waschen (verursacht durch den Gelbildner) neutralisieren, als auch nach dem Abtrocknen einen für den Verbraucher angenehmes Hautgefühl verursachen. Sie wirken nicht nur der Austrocknung durch die hohen Tensidmengen entgegen, sondern verbessern die Sensorik noch merklich.

Es kann auch gegebenenfalls vorteilhaft im Sinne der vorliegenden Erfindung sein, den Zubereitungen gemäß der Erfindung Farbstoffe und/oder -pigmente einzuverleiben.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe *Colour Index, 3. Auflage, Society of Dyers and Colourists, Bradford, England, 1971* entnommen.

Vorteilhaft im Sinne der vorliegenden Erfindung sind ferner Zubereitungen mit einem Gehalt an Perlglanzpigmenten. Bevorzugt sind insbesondere die im folgenden aufgelisteten Arten von Perlglanzpigmenten:
Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
Monokristalline Perlglanzpigmente wie z. B. Bismuthoxychlorid (BiOCl)
Schicht-Substrat Pigmente: z. B. Glimmer / Metalloxid
Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung** / **Schichtdicke** | **Farbe** |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 - 100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ | Goldtöne |
| | TiO₂ /Cr₂O₃ | grün |
| | TiO₂ / Berliner Blau | tiefblau |
| | TiO₂ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40-180 µm zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 20 Gew.-%, insbesondere von 1,0 bis 15 Gew.-% gewählt, jeweils bezogen auf das Gesamtgewicht der Zubereitungen.

Vorteilhaft, wenn auch nicht zwingend, können die erfindungsgemäßen Zubereitungen Konservierungsmittel enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.
Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Methodenbeschreibung

### Viskosität

Die Messung der Viskosität erfolgt bei 25°C in einem DIN Zylinder-System (Kegel-Platte System mit einem Durchmesser von 50mm) bei einer Scherrate von 100 1/s mit Hilfe eines Messgerätes mit der Bezeichnung Ares 8 aus der Rheometrix-Serie der Firma TA Instruments.

### Schaum

Die Schaumeigenschaften der Zubereitung ließen sich dabei wie folgt bestimmen:
Die Schaummessungen wurden mit einer modifizierten Variante des Ross-Miles Testes (DIN 53 902) durchgeführt. Diese Abwandlung des Testes wurde von Dr. F.J. Gohlke (Hoechst AG, Frankfurt) in der Zeitschrift Parfümerie und Kosmetik Nr.3/1964, Seiten 59 - 63 beschrieben. Hierbei wird die Versuchsanordnung leicht modifiziert, wodurch sich im
Vergleich zur DIN-Vorschrift etwas niedrigere Schaumhöhen ergeben.

Die Modifizierung sieht folgenden Versuchsaufbau vor:
50 ml der zu testenden Tensidlösung, auf 38° temperiert, werden in einen Standzylinder vorgelegt. Aus einer definierten Höhe wird 200 ml Wasser, ebenfalls auf 38° temperiert, mit einem Zusatz von 300 mg/l Calciumsulfat auf die Tensidlösung fallen gelassen. Die hierdurch erzeugte Schaummenge wird sofort, nach 30 Sekunden und nach 1, 3, 5, 10 bzw. 15 Minuten abgelesen und in ein Diagramm eingetragen. Aus den aufgenommenen Daten wird zusätzlich die Schaumstabilität errechnet und ebenfalls grafisch aufgetragen.

### Trübung

Das Produkt wird im Verhältnis von 1:5000 und 1:3000 mit 38°C warmem Wasser verdünnt. Dannach erfolgt die Messung der Transmission mit dem UV-Spekrotmeter 8453 der Firma HP in einer 1 cm Küvette (Plastikküvette der Firma Plastibrand, Katalog-Nr. 759170) bei einer Wellenlänge von 420 nm. Der Trübungsindex gibt die Transmission in % bei einer Wellenlänge von 420 nm bei einer Verdünnung von 1:5000 an.

Um die rückfettenden Eigenschaften der Zubereitung zu bestimmen, wurde ein Waschtest mit anschließender Lipidanalytik durchgeführt. Die rückfettenden Eigenschaften können dabei durch die Untersuchung der Lipidrückstände auf der Haut bestimmt werden.
Die Stabilitätseigenschaften der Zubereitung ließen sich dabei wie folgt bestimmen:
Als stabil wird eine Formel angesehen, wenn es nach Lagerung bei 40°C über einen Zeitraum von 2 Monaten nicht zur Wasser- und/oder Ölabscheidung kommt.

### Beispiele

Folgende Stoffe wurden eingesetzt:

| | |
|---|---|
| Polyacrylat: | Carbopol ETD 2020, Noveon |
| Natriumlaurethsulfat: | Hier wurde Texapon N 70 von der Firma Cognis verwendet. Gleiche Ergebnisse werden auch mit den Rohstoffen Genapol LRO Paste von Clariant und Emal 270 D/B von Kao erzielt. |
| Cocamidopropylbetain: | Tego-Betain F 50, Goldschmidt |
| Natrium Myristylethsulfat: | Texapon K 14 Spezial 70%, Cognis |
| Alkylpolyglucosid: | Hier kam Plantacare 2000 UP der Firma Cognis zum Einsatz. Ähnliche Ergebnisse werden auch mit Plantacare 1200 UP ebenfalls von Cognis erreicht. |
| PEG-7 Glyceryl Cocoate | Tegosoft GC von Goldschmidt kommt hier zum Einsatz. Auch Cetiol HE von Cognis und Glycerox HE B von Croda können eingesetzt werden. |
| Dimethicon | Hier wird das DC 200 Fluid 100 von Dow Corning verwendet. Der Rohstoff 0108055 Belsil DM 100 von Wacker erzielt die gleichen Ergebnisse. |
| Stearin Alkohol | Lanette 18, Cognis |
| Lanolin Alkohol | Eucerit PA, Beiersdorf |
| Farbstoffe | In den Beispielen 2 und 5 wurde der Farbstoff FD&C Blue No.1 von der BASF verwendet. |
| Effektpigmente | In den Beispielen 1 und 3 wurde das Pigment Timiron Gleamer Flake MP-45, Art. 117224 der Firma Merck verwendet. |

Die in der Rezeptur angegebenen Gehalte sind die Aktivgehalte der jeweiligen Rohstoffe.

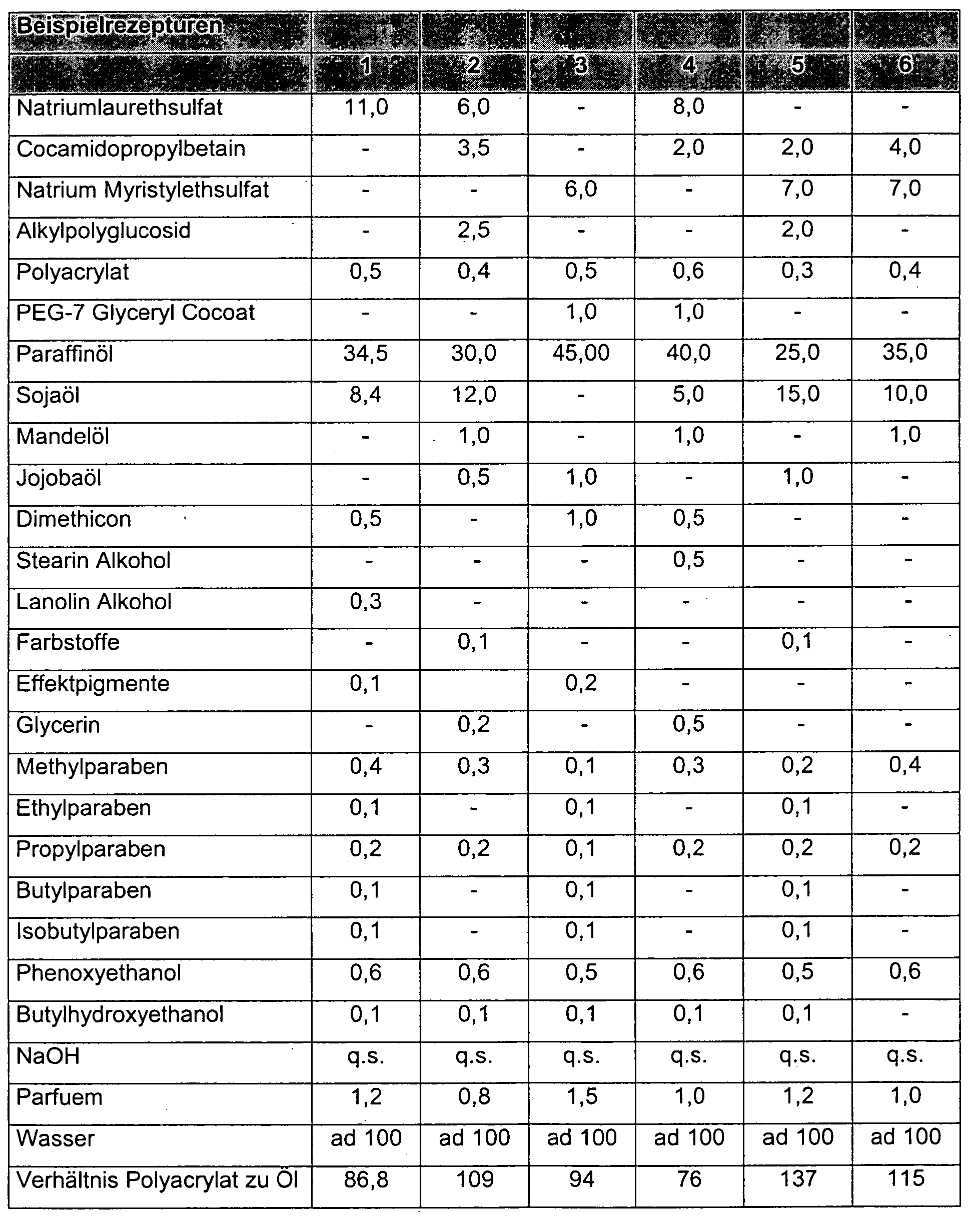

Die angegebenen Rohstoffe geben nur eine Auswahl der Möglichkeiten.

## Patentansprüche

1. Kosmetische Reinigungsemulsion enthaltend:
a) 41 - 51 %, besonders bevorzugt 43-47% Ölphase,
b) 2 - 17%, besonders bevorzugt 6-12% eines anionischen Tensids und
c) 0,1 bis 0,6%, besonders bevorzugt 0,3-0,6% eines Polyacrylates enthält,
wobei das Gewichtsverhältnis der Gesamtmenge Polyacrylat und der Gesamtmenge an Ölen vorteilhaft im Verhältnis von von 1:135 bis 1:85 und bevorzugt von 1:115 bis 1:95 gewählt wird und die Zubereitung eine Viskosität von 250 - 500 mPas bei 100 1/s aufweist, gemessen mit Ares 8 aus der Rheometrix-Serie der Firma TA Instruments bei 25°C in einem DIN Zylinder-System (Kegel-Platte System mit einem Durchmesser von 50mm) bei einer Scherrate von 100 1/s.

2. Zubereitung nach Patentanspruch 1 **dadurch gekennzeichnet, dass** sie im Wasser nicht deemulgiert.

3. Zubereitung nach einem der vorangehenden Patentansprüche **dadurch gekennzeichnet, dass** der Trübungsindex kleiner 10%, besonders bevorzugt kleiner 5% ist.

4. Zubereitung nach einem der vorangehenden Patentansprüche **dadurch gekennzeichnet, dass** das Mindestschaumvolumen nach der Ross-Miles Methode bei 100 - 140 ml liegt, besonders bevorzugt 110 - 130 ml.

5. Zubereitung nach einem der vorangehenden Patentansprüche **dadurch gekennzeichnet, dass** sie ein oder mehrere Öle aus der Gruppe der Kohlenwasserstoffe, der Fettsäuretriglyceride, Carbonsäureester enthält.

6. Zubereitung nach einem der vorangehenden Patentansprüche **dadurch gekennzeichnet, dass** das Verhältnis von Kohlenwasserstoffen zu Triglyceriden von 6:1 bis 2:1 und besonders bevorzugt von 5:1 bis 3:1 liegt.

7. Zubereitung nach einem der vorangehenden Patentansprüche **dadurch gekennzeichnet, dass** das anionische Tensid Natriumlaurethsulfat, Natriumparethsulfat und/oder Natriummyrethsulfat oder Mischungen aus den vorgenannten Substanzen ist.

8. Verwendung von Zubereitungen nach einem der vorangehenden Patentansprüche zur Reinigung und Pflege von menschlicher oder animalischer Haut oder Haar.

9. Verwendung von Zubereitungen nach einem der vorangehenden Patentansprüche als Wannenbad.
